# EUROPEAN PATENT APPLICATION

(11) **EP 4 299 752 A1**
(43) Date of publication of application: **03.01.2024**
(21) Application number: 22897056.2
(22) Date of filing: 10.05.2022
(51) Int. Cl.: C12P 7/10, C12M 1/40, C12M 1/00

(54) **SYSTEM AND METHOD FOR PRODUCING CELLULOSIC ETHANOL BY MEANS OF SACCHARIFICATION AND FERMENTATION OF BIOMASS**

(30) Priority: 25.11.2021 CN 202111416152
(71) Applicant: Zhejiang Huakang Pharmaceutical Co., Ltd., Quzhou, Zhejiang 324302 (CN)
(72) Inventor: HU, Changhui, Quzhou City, Zhejiang 324302 (CN); LUO, Jiaxing, Zhejiang 324302 (CN); LI, Mian, Santa Clara, California 95054 (US); LIAO, Chengjun, Quzhou City, Zhejiang 324302 (CN); YANG, Wulong, Quzhou City, Zhejiang 342302 (CN); YAN, Liangcong, Quzhou City, Zhejiang 324302 (CN); ZHEN, Ni, Quzhou City, Zhejiang 324302 (CN); CHEN, Lanlan, Quzhou City, Zhejiang 324302 (CN)
(74) Representative: Sun, Yiming
(86) International application number: PCT/CN2022/092087
(87) International publication number: WO 2023/092956

(57) **Abstract**

The present disclosure provides a system for producing cellulosic ethanol by biomass saccharification and fermentation. The system includes a reactor A configured to carry out an enzymatic hydrolysis reaction of a biomass feedstock, a reactor B configured to carry out a fermentation reaction of the biomass feedstock, and a collection tank configured to collect the cellulosic ethanol produced from the fermentation reaction of the biomass feedstock. The reactor A is connected to the reactor B, the storage tank, and the collection tank, and the reactor B is connected to the storage tank. The present disclosure also provides a method for preparing cellulosic ethanol implemented by the system, by which multiple cycles processes of enzymatic hydrolysis, fermentation, and the ethanol extraction are carried out on the biomass feedstock at intervals, thereby reducing the inhibitory effect of the ethanol accumulation on the hydrolysis and fermentation processes and improving the conversion rate of the cellulosic ethanol. By setting a storage tank, two batches of materials can be processed simultaneously in one system, thereby greatly improving the production efficiency of cellulosic ethanol.

## Description

### TECHNICAL FIELD

The present disclosure relates to a technical field of cellulosic ethanol production, and in particular to systems and methods for producing cellulosic ethanol by biomass saccharification and fermentation.

### BACKGROUND

The development of sustainable and pollution-free energy sources is a technological development trend. A large amount of lignocellulosic waste such as straw and corncobs are produced every year in agricultural production. Main components in lignocellulosic material are cellulose, hemicellulose, lignin, and a small amount of ash. The cellulose may be hydrolyzed by cellulase to obtain glucose. The glucose may be used to produce cellulosic ethanol through biological fermentation. The cellulosic ethanol may be used as a clean fuel.

At present, there are many bottlenecks in a process of producing the cellulosic ethanol from a biomass feedstock. The process of producing cellulosic ethanol mainly includes pretreatment of the biomass feedstock, cellulase enzymatic hydrolysis, and glucose fermentation. For the cellulase enzymatic hydrolysis and saccharification, the challenge is an inhibitory effect of produced glucose in a process of increasing a high solid content of an enzymatic hydrolysis system, especially in a later stage of enzymatic hydrolysis, a concentration of released monomeric glucose increases, which exerts a stronger inhibitory effect on cellulase activity, thereby leading to a decrease in overall enzymatic hydrolysis efficiency and in conversion efficiency of cellulose by enzymatic hydrolysis.

To address the issue of the product inhibitory during enzymatic hydrolysis, a most widely used process is simultaneous saccharification and fermentation (SSF). The SSF enables microbial fermentation of glucose into cellulosic ethanol when the glucose is released during the cellulase enzymatic hydrolysis, thus alleviating the product inhibitory. The cellulase enzymatic hydrolysis and the glucose fermentation are combined into one process. However, there are still some problems. At present, an optimal reaction temperature for common and efficient commercial cellulase is usually above 45°C, while an optimal fermentation temperature for common ethanol fermentation strains, such as brewing yeast, is 35°C. At 45°C, the ethanol fermentation strains not only cannot grow to produce ethanol, but also tend to apoptosis. The SSF process cannot ensure the optimal conditions for the enzymatic saccharification and the fermentation at the same time. Besides, the produced cellulosic ethanol has a certain inhibitory effect on the cellulase, thereby affecting the enzymatic hydrolysis and fermentation efficiency.

### SUMMARY

The technical problem that the present disclosure aims to solve is to provide a system and a method for producing cellulosic ethanol by biomass saccharification and fermentation, which integrates the cellulase hydrolysis process and the ethanol fermentation process into an intermittent hydrolysis and fermentation process to address the issue of the activity inhibition of the cellulase caused by the produced glucose, thereby improving the production of the cellulosic ethanol.

To achieve this, the present disclosure provides a system for producing cellulosic ethanol by biomass saccharification and fermentation. The system includes a reactor A, a reactor B, and a collection tank. The reactor A is configured to carry out an enzymatic hydrolysis reaction of a biomass feedstock. The reactor B is configured to carry out a fermentation reaction of the biomass feedstock. The collection tank is configured to collect the cellulosic ethanol produced from the fermentation reaction of the biomass feedstock. An enzymatic hydrolysis dosing pipe and a fermentation dosing pipe are respectively provided on the reactor A and the reactor B. An outlet of the reactor A is connected to an inlet of the reactor B through a fermentation pipe, and a diaphragm pump A is provided on the fermentation pipe. An outlet of the reactor B is connected to an inlet of the reactor A through a discharge pipe, a diaphragm pump B and a first discharge pipe are provided on the discharge pipe, and the first discharge pipe is located behind the diaphragm pump B. An upper part of the reactor A is connected to an inlet of the collection tank through an ethanol pipe. An insulation layer is provided outside the reactor A for heating and insulation. A coil insulation device is provided at a connection of the ethanol pipe and the reactor A, the coil insulation device and the insulation layer are respectively connected to a hot water tank through a hot water pipe, and a centrifugal pump is provided on the hot water pipe.

Specifically, the enzymatic hydrolysis dosing pipe is configured to add a hydrolyzable biomass feedstock, acid, and hydrolysis material etc., to the reactor A. The fermentation dosing pipe is configured to add fermentation material such as fermentation strains to the reactor B. The biomass feedstock includes straw and agricultural residues, and common biomass feedstock include corn straw, corn cob, bagasse, etc. The ethanol pipe is configured to transport cellulosic ethanol vapors evaporated from fermented biomass feedstock in the reactor A.

Further, a storage tank, a diaphragm pump C, and a second discharge pipe are provided on the discharge pipe. The storage tank is located behind the first discharge pipe and configured to store material discharged from the reactor B, and the second discharge pipe is located behind diaphragm pump C.

The present disclosure provides a method for producing cellulosic ethanol by biomass saccharification and fermentation. The method is implemented by the system for producing cellulosic ethanol by biomass saccharification and fermentation as described above. The method includes the following steps 1-5.

In step 1, the biomass feedstock with 15%-20% of a dry substrate is added to a citric acid buffer solution with a concentration of 0.1 mol/L, and then cellulase with 4% -6% of the dry substrate is added to form a mixture. A pH of the citric acid buffer solution is maintained at 5.0~6.0. Further, the prepared mixture is dosed to the reactor A through the enzymatic hydrolysis dosing pipe.

In step 2, a temperature of the reactor A is maintained at an enzymatic hydrolysis reaction temperature T, the pH value of the material (i.e., the mixture) in the reactor A is maintained, and a first enzymatic hydrolysis reaction of the material is carried out in the reactor A for 10~14 hours under stirring.

In step 3, the material after the enzymatic hydrolysis reaction in the reactor A is transported to the reactor B through the fermentation pipe, and then fermentation strains with a mass ratio of 0.1% - 1% are added to the reactor B through the fermentation dosing pipe. Further, a fermentation temperature is maintained at a suitable fermentation temperature for the fermentation strains, and a first fermentation reaction of the material is carried out in the reactor B for 10~14 hours under stirring.

In step 4, after the first fermentation reaction is completed, the material after the fermentation reaction in the reactor B is transported to the reactor A through the discharge pipe. The cellulosic ethanol in the material produced by the fermentation is evaporated into cellulosic ethanol vapors in the reactor A, and the cellulosic ethanol vapors flow into the collection tank through the ethanol pipe and are condensed into the cellulosic ethanol.

In step 5, the steps 2-4 are repeated. A same batch of material is carried out a single-circulation for 4~6 cycles respectively in the reactor A and the reactor B, and after the circulation is completed in the reactor B, the material is discharged from the system through the first discharge pipe.

The present disclosure provides another method for producing cellulosic ethanol by biomass saccharification and fermentation. The method is implemented by the system for producing cellulosic ethanol by biomass saccharification and fermentation as described above. The method includes the following steps I-V.

In step I, the biomass feedstock with 15%-20% of a dry substrate is added to a citric acid buffer solution with a concentration of 0.1 mol/L, and then cellulase with 4% -6% of the dry substrate is added to form a mixture. A pH of the citric acid buffer solution is maintained at 5.0~6.0. Further, the prepared first batch of material (i.e., the mixture) is dosed to the reactor A through the enzymatic hydrolysis dosing pipe.

In step II, a temperature of the reactor A is maintained at an enzymatic hydrolysis reaction temperature T, the pH value of the material in the reactor A is maintained, and a first enzymatic hydrolysis reaction of the first batch of material is carried out in the reactor A for 10~14 hours under stirring.

In step III, the first batch of material after the enzymatic hydrolysis reaction in the reactor A is transported to the reactor B through the fermentation pipe, and then fermentation strains with a mass ratio of 0.1 % - 1% are added to the reactor B through the fermentation dosing pipe. Further, a fermentation temperature is maintained at a suitable fermentation temperature for the fermentation strains, and a first fermentation reaction of the first batch of material is carried out in the reactor B for 10~14 hours under stirring.

After the first batch of material after the enzymatic hydrolysis reaction in the reactor A has been emptied, the steps I and II are repeated, a second batch of material is added to the reactor A, and the enzymatic hydrolysis reaction of the second batch of material is carried out in the reactor A.

In step IV, after the first fermentation reaction is completed, the first batch of material in the reactor B is transported to the storage tank through the discharge pipe for temporary storage, and after the first batch of material in the reactor B is emptied, the step III is repeated to transport the second batch of material in the reactor A to the reactor B through the fermentation pipe for the fermentation reaction.

In step V, after the second batch of material in the reactor A is emptied, the first batch of material in the storage tank is transported to the reactor A through the discharge pipe, and the step II is repeated. The cellulosic ethanol in the first batch of material produced by the fermentation is evaporated into the cellulosic ethanol vapors in the reactor A, and the cellulosic ethanol vapors are transported into the collection tank through the ethanol pipe and condensed into the cellulosic ethanol.

After the first batch of material in the storage tank is emptied, the step IV is repeated to transport the second batch of material in the reactor B to the storage tank through the discharge pipe for temporary storage.

In step VI, the steps II-V are repeated. The two batches of material are carried out a double-circulation, the circulation of each of the two batches of material is carried out for 4~6 cycles respectively in the reactor A and the reactor B, and after the circulations of the two batches of material are completed in the reactor B in sequence, the two batches of material are discharged from the system in sequence through the first discharge pipe.

Compared with the prior art, the system and the methods for producing cellulosic ethanol by biomass saccharification and fermentation in the present disclosure have the following features.
1. During high-solid enzymatic hydrolysis and fermentation of the biomass feedstock, the methods of the present disclosure can use the fermentation strains to ferment glucose into cellulosic ethanol after a hydrolysis product (i.e., fermentable glucose) has accumulated to a certain concentration, thereby solving the problem of glucose accumulation inhibiting cellulase activity.
2. By combining the cellulase hydrolysis process, the fermentation process, and the ethanol extraction process, these three processes are combined into a cyclic process in which the biomass feedstock are subjected to multiple cycles of the enzymatic saccharification, the fermentation, and the ethanol extraction at intervals, which reduces the inhibition of ethanol accumulation on the hydrolysis and fermentation processes, thereby increasing the conversion rate of the cellulosic ethanol.

### BRIEF DESCRIPTION OF THE DRAWINGS

FIG. 1 is a schematic diagram illustrating an exemplary structure of a system for producing cellulosic ethanol by biomass saccharification and fermentation according to some embodiments of the present disclosure;
FIG. 2 is a schematic diagram illustrating an exemplary structure of the system for producing cellulosic ethanol by biomass saccharification and fermentation according to some embodiments of the present disclosure;
FIG. 3 is a schematic diagram illustrating that glucose release concentration changes over time in an enzymatic hydrolysis system with external addition of glucose and ethanol according to some embodiments of the present disclosure;
FIG. 4 is a columnar schematic diagram illustrating survival rates of brewing yeast under different temperatures in a heat resistance experiment according to some embodiments of the present disclosure; and
FIG. 5 is a columnar schematic diagram illustrating survival rates of Zymomonas mobilis under different temperatures in a heat resistance experiment according to some embodiments of the present disclosure.

### DETAILED DESCRIPTION

In order to make the technical problems, technical solutions, and beneficial effects to be solved by the present invention clearer and more comprehensible, the present disclosure will be further described in detail in conjunction with drawings and embodiments. It should be understood that the specific embodiments described herein are for the purpose of explaining the present disclosure and are not intended to limit the present disclosure.

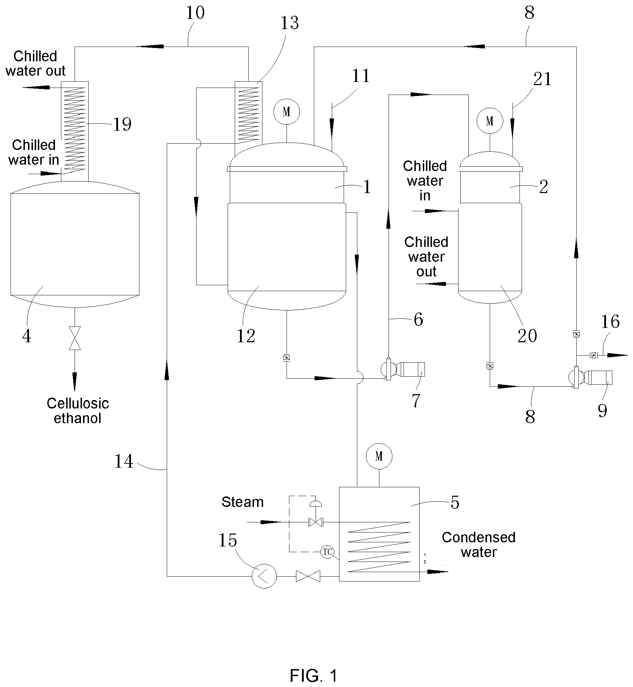

FIG. 1 shows a first embodiment of a system for producing cellulosic ethanol by biomass saccharification and fermentation in the present disclosure. Arrows in the figure indicate directions of travel of materials in the system, e.g., a biomass feedstock, steam, chilled water, condensed water, strains, ethanol, etc. The system includes a reactor A1, a reactor B, and a collection tank 4. The reactor A1 is configured to carry out an enzymatic hydrolysis reaction of a biomass feedstock. The reactor B is configured to carry out a fermentation reaction of the biomass feedstock. The collection tank 4 is configured to collect the cellulosic ethanol produced from the fermentation reaction of the biomass feedstock.

An enzymatic hydrolysis dosing pipe 11 and a fermentation dosing pipe 21 are respectively provided on the reactors A1 and on the reactor B2, and an outlet of the reactor A1 is connected to an inlet of the reactor B2 through a fermentation pipe 6. A diaphragm pump A7 is provided on the fermentation pipe 6, and an outlet of the reactor B2 is connected to an inlet of the reactor A1 through a discharge pipe 8. A diaphragm pump B9 and a first discharge pipe 16 are provided on the discharge pipe 8, and the first discharge pipe 16 is located behind the diaphragm pump B9. An upper part of the reactor A is connected to an inlet of the collection tank 4 through an ethanol pipe 10.

An insulation layer 12 for heating and insulation of the reactor A1 is provided outside the reactor A1, and a coil insulation device 13 is provided at a connection of the reactor A1 and the ethanol pipe 10 located at the upper part of the reactor A1. The coil insulation device 13 and the insulation layer 12 are respectively connected to a hot water tank 5 through a hot water pipe 14, and a centrifugal pump 15 is provided on the hot water pipe 14. The coil insulation device 13 is configured to ensure that the cellulosic ethanol vapors evaporated in the reactor A1 do not condense and are transported to the collection tank 4. Hot water in the hot water tank 5 enters the coil insulation device 13 located at the upper part of the reactor A1 and the insulation layer 12 outside the reactor A1 through the centrifugal pump 15 to control the temperatures of the reactor A1 and the coil insulation device 13.

An ethanol freezing device 19 configured to condense the cellulosic ethanol vapors is provided at a connection of the collection tank 4 and the ethanol pipe 10 located at a top of the collection tank 4. The ethanol freezing device 19 facilitates the condensation and collection of the cellulosic ethanol vapors passing through the ethanol pipe 10 by chilled water.

A fermentation cooling device 20 configured to cool fermentation material inside the reactor B2 is provided outside the reactor B2.

A stirring device (not shown in the figure) is provided in the reactors A1 and in the B2, respectively.

In the first embodiment, a same batch of biomass material is carried out a single-circulation flow between the reactor A1 and the reactor B2.

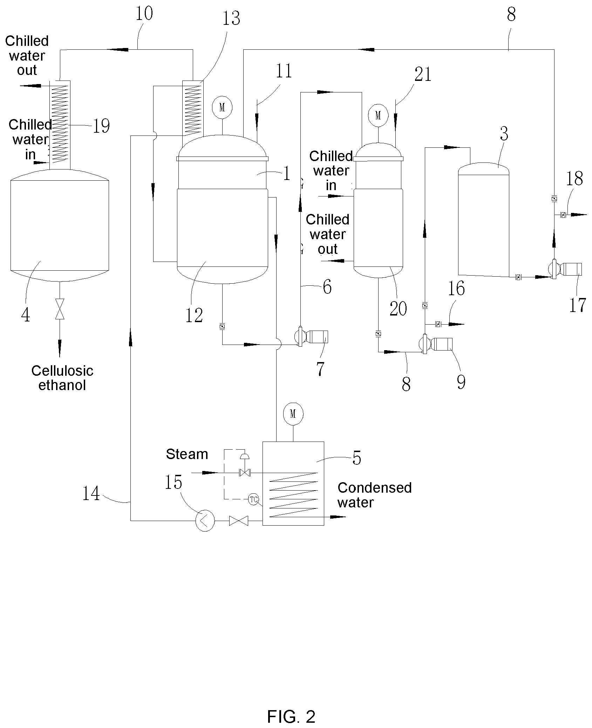

FIG. 2 shows a second embodiment of the system for producing cellulosic ethanol by biomass saccharification and fermentation in the present disclosure. Compared with the first embodiment, in the second embodiment, a storage tank 3, a diaphragm pump C17, and a second discharge pipe 18 are provided on the discharge pipe 8. The storage tank 3 is located behind the first discharge pipe 16 and is configured to store material discharged from the reactor B2. The second discharge pipe 18 is located behind the diaphragm pump C17. Other structures are the same as in the first embodiment, which are not be repeated here.

In the second embodiment, due to the addition of the storage tank 3 as a turnover temporary storage tank, two batches of biomass material may be allowed to circulate simultaneously in the system. That is, the two batches of biomass material are carried out a double-circulation flow in the reactor A1 and in the reactor B2 in sequence.

The present disclosure also discloses a method for producing cellulosic ethanol by biomass saccharification and fermentation. The method is implemented by the system for producing cellulosic ethanol by biomass saccharification and fermentation as described above. The method includes the following steps 1-5.

In step 1, the biomass feedstock with 15%-20% (w/w) of a dry substrate is added to a citric acid buffer solution with a concentration of 0.1 mol/L, and then cellulase with 4% -6% (w/w) of the dry substrate is added to form a mixture. A pH of the citric acid buffer solution is maintained at 5.0~6.0. Further, the prepared mixture is dosed to the reactor A1 through the enzymatic hydrolysis dosing pipe 11.

In step 2, a temperature of the reactor A1 is maintained at an enzymatic hydrolysis reaction temperature T, the pH value of the material (i.e., the mixture) in the reactor A is maintained, and a first enzymatic hydrolysis reaction of the material in the reactor A is carried out for 10~14 hours under stirring.

In step 3, the material after the enzymatic hydrolysis reaction in the reactor A1 is transported to the reactor B2 through the fermentation pipe 6, and then fermentation strains with a mass ratio of 0.1% - 1% are added to the reactor B2 through the fermentation dosing pipe 21. Further, a fermentation temperature is maintained at a suitable fermentation temperature for the fermentation strains, and a first fermentation reaction of the material is carried out in the reactor B2 for 10~14 hours under stirring.

In step 4, after the first fermentation reaction is completed, the material after the fermentation reaction in the reactor B2 is transported to the reactor A1 through the discharge pipe 8. The cellulosic ethanol in the material produced by the fermentation is evaporated into cellulosic ethanol vapors in the reactor A, and the cellulosic ethanol vapors flow into the collection tank through the ethanol pipe and are condensed into the cellulosic ethanol.

In step 5, the steps 2-4 are repeated. A same batch of material is carried out a single-circulation for 4~6 cycles respectively in the reactor A1 and the reactor B2 in which the enzymatic hydrolysis reaction and the fermentation reaction are respectively carried out 4~6 times, and after the circulation is completed in the reactor B2, the material is discharged from the system through the first discharge pipe 16.

This is a single-circulation approach of the method for producing cellulosic ethanol by biomass saccharification and fermentation.

The enzymatic hydrolysis reaction temperature T is a highest temperature at which a survival rate of the fermentation strains is above 80%. The enzymatic hydrolysis reaction temperature T is determined by preparing a test system by using the citric acid buffer solution with a concentration of 0.1 mol/L, adding glucose with a concentration of 100 g/L and the fermentation strains with a mass ratio of 1% in the test system, incubating the test system for 12h at 40°C, 41°C, 42°C, 43°C, 44°C, 45°C, 46°C, 47°C, 48°C, 49°C, 50°C, respectively, for the test system incubated at each of the 40°C, 41°C, 42°C, 43°C, 44°C, 45°C, 46°C, 47°C, 48°C, 49°C, and 50°C, measuring a count of viable bacteria of the fermentation strains using a plate counting technique, and selecting a highest temperature at which the survival rate of the viable bacteria is 80% after the 12 hours of incubation as the enzymatic hydrolysis reaction temperature T.

The cellulase in the enzymatic hydrolysis reaction is Cellic CTec 2.

The present disclosure also discloses another method for producing cellulosic ethanol by biomass saccharification and fermentation. The method is implemented by the system for producing cellulosic ethanol by biomass saccharification and fermentation as described above. The method includes the following steps I-V.

In step I, the biomass feedstock with 15%-20% of a dry substrate is added to a citric acid buffer solution with a concentration of 0.1 mol/L, and then cellulase with 4% -6% of the dry substrate is added to form a mixture. A pH of the citric acid buffer solution is maintained at 5.0~6.0. Further, the prepared first batch of material (i.e., the mixture) is dosed to the reactor A1 through the enzymatic hydrolysis dosing pipe 11.

In step II, a temperature of the reactor A1 is maintained at an enzymatic hydrolysis reaction temperature T, the pH value of the material in the reactor A1 is maintained, and a first enzymatic hydrolysis reaction of the first batch of material is carried out in the reactor A1 for 10~14 hours under stirring.

In step III, the first batch of material after the enzymatic hydrolysis reaction in the reactor A1 is transported to the reactor B2 through the fermentation pipe 6, and then fermentation strains with a mass ratio of 0.1 % -1 % are added to the reactor B2 through the fermentation dosing pipe 21. Further, a fermentation temperature is maintained at a suitable fermentation temperature for the fermentation strains, and a first fermentation reaction of the first batch of material is carried out in the reactor B2 for 10~14 hours under stirring.

After the first batch of material after the enzymatic hydrolysis reaction in the reactor A1 has been emptied, the steps I and II are repeated, a second batch of material is added to the reactor A, and the enzymatic hydrolysis reaction of the second batch of material is carried out in the reactor A.

In step IV, after the first fermentation reaction is completed, the first batch of material in the reactor B2 is transported to the storage tank 3 through the discharge pipe 8 for temporary storage, and after the first batch of material in the reactor B2 is emptied, the step III is repeated to transport the second batch of material in the reactor A1 to the reactor B2 through the fermentation pipe 6 for the fermentation reaction.

In step V, after the second batch of material in the reactor A1 is emptied, the first batch of material in the storage tank 3 is transported to the reactor A1 through the discharge pipe 8, and the step II is repeated. The cellulosic ethanol in the first batch of material produced by the fermentation is evaporated into the cellulosic ethanol vapors in the reactor A, and the cellulosic ethanol vapors are transported into the collection tank through the ethanol pipe and condensed into the cellulosic ethanol.

After the first batch of material in the storage tank 3 is emptied, the step IV is repeated to transport the second batch of material in the reactor B2 to the storage tank 3 through the discharge pipe 8 for temporary storage.

In step VI, the steps II-V are repeated. The two batches of material are carried out a double-circulation, the circulation of each of the two batches of material is carried out for 4~6 cycles respectively in the reactor A1 and the reactor B2, and after the circulations of the two batches of material are completed in the reactor B2 in sequence, the two batches of material are discharged from the system in sequence through the first discharge pipe 16.

Other steps that are the same as steps in the previous embodiment will not be described again.

This is a double-circulation approach of the method for producing cellulosic ethanol by biomass saccharification and fermentation.

The following embodiments are provided to further illustrate the system and method for producing cellulosic ethanol by biomass saccharification and fermentation in the present disclosure.

### Embodiment 1

Validation experiment of the inhibitory effect of glucose and ethanol on the enzymatic hydrolysis reaction.

A reaction system was prepared using a citric acid buffer solution with a concentration of 0.1 mol/L. The citric acid buffer solution was added to a corn cob waste system in which a content of the biomass feedstock was 15% of a dry substrate, a pH of the mixture was maintained at 5.0 ~ 6.0, and cellulase Cellic CTec 2 with a content of 4% of the dry substrate was added to the mixture to prepare three groups of 1L reaction systems.

| Groups | Externally added inhibitor |
|---|---|
| A | None |
| B | 100g/L glucose |
| C | 50g/L ethanol |

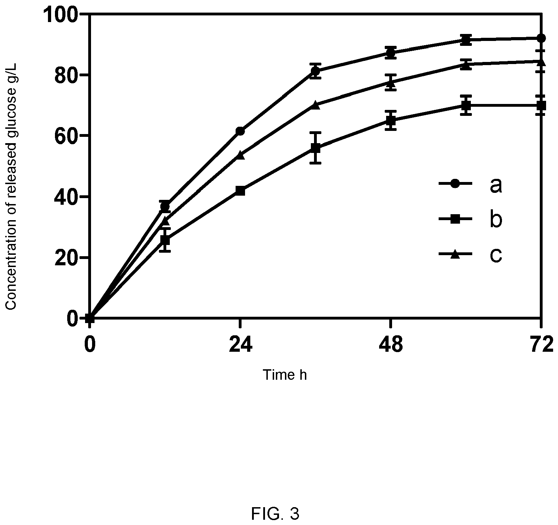

The three reaction systems were subjected to enzymatic hydrolysis at 50°C for 72 hours under a condition of stirring at 300rpm. Samples were taken from each reaction system every 12 hours to measure an amount of glucose released by the enzymatic hydrolysis, and results are shown in FIG. 3.

Experimental conclusion: both glucose and ethanol have certain inhibitory effects on the enzymatic hydrolysis of cellulose. After 100 g/L of glucose was added to Group B, the amount of glucose released during the 72-hour enzymatic hydrolysis was 24% lower than that of the control Group A without inhibitor. After 50 g/L of ethanol was added to Group C, the amount of glucose released during the 72-hour enzymatic hydrolysis was 8% lower than that of the control Group A.

### Embodiment 2

A heat resistance experiment was carried out on Angel brewing yeast and Zymomonas mobilis to determine the enzymatic hydrolysis reaction temperature T.

The method in the present disclosure is applicable to a variety of ethanol fermentation strains. This experiment is illustrated by taking two common ethanol fermentation strains: Angel brewing yeast and Zymomonas mobilis as examples.

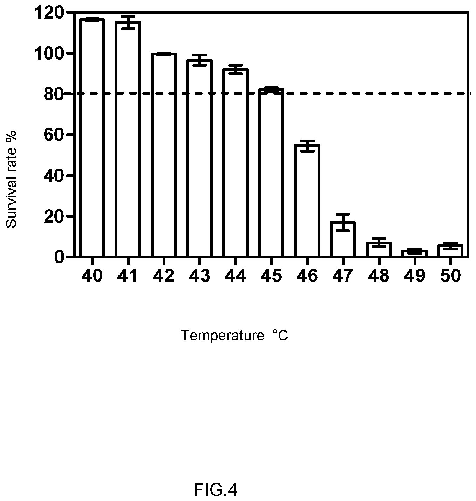

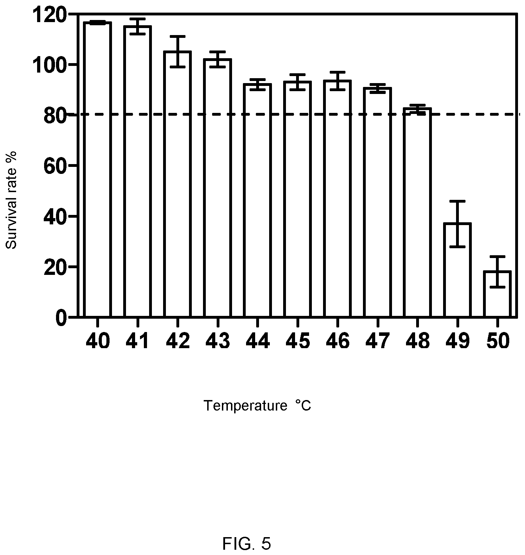

A 100g/L glucose solution was prepared using a citric acid buffering solution. The solution was divided into two portions. One portion was mixed with 1% mass concentration of Angel brewing yeast powder, and the other portion was mixed with 1% mass concentration of Zymomonas mobilis bacteria liquid. Samples were then taken from each portion. The samples were incubated at different temperatures (multiple temperatures within the range of 40°C~50°C, with an interval of 1 degree between adjacent temperatures) for 12 hours. For the sample incubated at each temperature, a count of viable bacteria before and after the incubation was measured using a plate counting technique, and a change in the count of viable bacteria before and after incubation was determined, and results are shown in FIG. 4 and FIG. 5.

Experimental conclusion: through statistical analysis, after 12 hours of incubation, a highest temperature at which a survival rate of the fermentation strains is above 80% was selected as the enzymatic hydrolysis reaction temperature T. The enzymatic hydrolysis reaction temperature T corresponding to the Angel brewing yeast was 45°C, and the enzymatic hydrolysis reaction temperature T corresponding to the Zymomonas mobilis was 48°C.

### Embodiment 3

A first single-circulation embodiment of the method for producing cellulosic ethanol by biomass saccharification and fermentation in the present disclosure includes steps 11-17.

In step 11, the enzymatic hydrolysis reaction temperature T was determined according to the above Embodiment 2.

In step 12, material of the reaction system was prepared by adding corn cob waste with a dry substrate content of 15kg (w/w) to an aqueous solution containing a citric acid buffer system to make the mixture reach 100kg, a pH of the mixture was maintained at 5.0~6.0, 0.6kg cellulase Cellic CTec 2 was added to the mixture, and the mixture was stirred evenly. Further, the prepared material was added into the first reactor A1 through the enzymatic hydrolysis dosing pipe 11.

In step 13, a temperature of the first reactor A1 was set to and maintained at 45°C, and the enzymatic hydrolysis reaction was carried out for 10-14 hours under stirring of 300rpm.

In step 14, a temperature of the second reactor B2 was set to 35°C (an optimal fermentation temperature of the brewing yeast), after the material after the enzymatic hydrolysis reaction in step 13 was transported from the first reactor A1 to the second reactor B2 through the diaphragm pump A7, 1kg of Angel brewing yeast powder was inoculated into the second reactor B2 through the fermentation dosing pipe 21 of the second reactor B2, and the fermentation reaction was carried out for 10~14 hours under stirring of 100rpm to complete a reaction cycle.

In step 15, after the material after the fermentation reaction in step 140 was transported from the second reactor B2 to the first reactor A1, a second enzymatic hydrolysis reaction was carried out under constant stirring, and produced cellulosic ethanol was evaporated and collected in the collection tank 4.

In step 16, the material after the second enzymatic hydrolysis in step 150 was transported from the first reactor A1 to the second reactor B2.

In step 17, steps 14 to 16 were repeated for 5 cycles.

Finally, 4.4kg of material in the collection tank 4 was obtained, which contained 35% cellulosic ethanol, and the amount of the obtained cellulosic ethanol was 1.54kg. Material in the second reactor B2 was 89.5kg, in which the cellulosic ethanol content was 3.1%, and the amount of the obtained cellulosic ethanol was 2.77kg. A total amount of the obtained cellulosic ethanol was 4.31kg, and a conversion rate of the cellulosic ethanol was 84.5%.

### Embodiment 4

A second single-circulation embodiment of the method for producing cellulosic ethanol by biomass saccharification and fermentation in the present disclosure includes steps 21-27.

In step 21, the enzymatic hydrolysis reaction temperature T was determined according to the above Embodiment 2.

In step 22, material of the reaction system was prepared by adding corn cob waste with a dry substrate content of 20kg (w/w) to an aqueous solution containing a citric acid buffer system to make the mixture reach 100kg, a pH of the mixture was maintained at 5.0~6.0, 0.8kg cellulase Cellic CTec 2 was added to the mixture, and the mixture was stirred evenly. Further, the prepared material was added into the first reactor A1 through the enzymatic hydrolysis dosing pipe 11.

In step 23, a temperature of the first reactor A1 was set to and maintained at 45°C, and the enzymatic hydrolysis reaction was carried out for 10-14 hours under stirring of 300rpm.

In step 24, a temperature of the second reactor B2 was set to 35°C, after the material after the enzymatic hydrolysis reaction in step 23 was transported from the first reactor A1 to the second reactor B2 through the diaphragm pump A7, 1kg of Angel brewing yeast powder was inoculated into the second reactor B2 through the fermentation dosing pipe 21 of the second reactor B2, and the fermentation reaction was carried out for 10~14 hours under stirring of 100rpm to complete a reaction cycle.

In step 25, after the material after the fermentation reaction in step 24 was transported from the second reactor B2 to the first reactor A1, a second enzymatic hydrolysis reaction was carried out under constant stirring, and produced cellulosic ethanol was evaporated and collected in the collection tank 4.

In step 26, the material after the second enzymatic hydrolysis in step 25 was transported from the first reactor A1 to the second reactor B2.

In step 27, steps 24 to 26 were repeated for 6 cycles.

Finally, 4.7kg of material in the collection tank 4 was obtained, which contained 38% cellulosic ethanol, and the amount of the obtained cellulosic ethanol was 1.79kg. Material in the second reactor B2 was 90.5kg, in which the cellulosic ethanol content was 4.1%, and the amount of the obtained cellulosic ethanol was 3.71kg. A total amount of the obtained cellulosic ethanol was 5.50kg, and a conversion rate of the cellulosic ethanol was 80.9%.

### Embodiment 5

A third single-circulation embodiment of the method for producing cellulosic ethanol by biomass saccharification and fermentation in the present disclosure includes steps 31-37.

In step 31, the enzymatic hydrolysis reaction temperature T was determined according to the above Embodiment 2.

In step 32, material of the reaction system was prepared by adding corn cob waste with a dry substrate content of 20kg (w/w) to an aqueous solution containing a citric acid buffer system to make the mixture reach 100kg, a pH of the mixture was maintained at 5.0~6.0, 0.8kg cellulase Cellic CTec 2 was added to the mixture, and the mixture was stirred evenly. Further, the prepared material was added into the first reactor A1 through the enzymatic hydrolysis dosing pipe 11.

In step 33, a temperature of the first reactor A1 was set to and maintained at 48°C, and the enzymatic hydrolysis reaction was carried out for 10-14 hours under stirring of 300rpm.

In step 34, a temperature of the second reactor B2 was set to 35°C, after the material after the enzymatic hydrolysis reaction in step 33 was transported from the first reactor A1 to the second reactor B2 through the diaphragm pump A7, 1kg of activated Zymomonas mobilis bacteria liquid was inoculated into the second reactor B2 through the fermentation dosing pipe 21 of the second reactor B2, and the fermentation reaction was carried out for 10~14 hours under stirring of 100rpm to complete a reaction cycle.

In step 35, after the material after the fermentation reaction in step 34 was transported from the second reactor B2 to the first reactor A1, a second enzymatic hydrolysis reaction was carried out under constant stirring, and produced cellulosic ethanol was evaporated and collected in the collection tank 4.

In step 36, the material after the second enzymatic hydrolysis in step 35 was transported from the first reactor A1 to the second reactor B2.

In step 37, steps 34 to 36 were repeated for 6 cycles.

Finally, 4.8kg of material in the collection tank 4 was obtained, which contained 39% cellulosic ethanol, and the amount of the obtained cellulosic ethanol was 1.87kg. Material in the second reactor B was 93.5kg, in which the cellulosic ethanol content was 4.13%, and the amount of the obtained cellulosic ethanol was 3.86kg. A total amount of the obtained cellulosic ethanol was 5.73kg, and a conversion rate of the cellulosic ethanol was 84.3%.

### Embodiment 6

A first double-circulation embodiment of the method for producing cellulosic ethanol by biomass saccharification and fermentation in the present disclosure includes steps 41-48.

In step 41, the enzymatic hydrolysis reaction temperature T was determined according to the above Embodiment 2.

In step 42, a first batch of material of the reaction system was prepared by adding corn cob waste a dry substrate content of 20kg (w/w) to an aqueous solution containing a citric acid buffer system to make the mixture reach 100kg, a pH of the mixture was maintained at 5.0~6.0, 0.8kg cellulase Cellic CTec 2 was added to the mixture, and the mixture was stirred evenly. Further, the prepared first batch of material was added into the first reactor A1 through the enzymatic hydrolysis dosing pipe 11.

In step 43, a temperature of the first reactor A1 was set to and maintained at 45°C, and the enzymatic hydrolysis reaction was carried out for 10-14 hours under stirring of 300rpm.

In step 44, a temperature of the second reactor B2 was set to 35°C, after the first batch of material after the enzymatic hydrolysis reaction in step 43 was transported from the first reactor A1 to the second reactor B2 through the diaphragm pump A7, 1kg of Angel brewing yeast powder was inoculated into the second reactor B2 through the fermentation dosing pipe 21 of the second reactor B2, and the fermentation reaction was carried out for 10~14 hours under stirring of 100rpm to complete a reaction cycle of the first batch of material.

After the first batch of material was transported from the first reactor A1 to the second reactor B2 through the diaphragm pump A7, a second batch of material of the reaction system was prepared according to the preparation manner in step 42, and the second batch of material of the reaction system was processed according to step 43, i.e., the second batch of material prepared according to step 42 was added to the first reactor A1 through the enzymatic hydrolysis dosing pipe 11, and the enzymatic hydrolysis reaction was carried out for 10~14 hours under stirring of 300rpm.

In step 45, the first batch of material in the second reactor B2 was transported to the storage tank 3 through the diaphragm pump B9, and then the second batch of material in the first reactor A1 was transported to the second reactor B2, and step 44 is performed on the second batch of material to complete a reaction cycle of the second batch of material.

In step 46, the first batch of material in the storage tank 3 was transported to the first reactor A1 through the discharge pipe 8 and continuously stirred, and produced cellulosic ethanol was evaporated and collected in the collection tank 4, while the second batch of material was transported from the second reactor B2 to the storage tank 3.

In step 47, the first batch of material was again transported from the first reactor A1 to the second reactor B2. The second batch of material was always one container behind the first batch of material.

In step 48, steps 44-46 were repeated for 6 cycles.

Finally, 8.8kg of material in the collection tank 4 was obtained, which contained 38.5% cellulosic ethanol, and the amount of the obtained cellulosic ethanol was 3.39kg. A total of 178.5 kg of the two batches of material were discharged, in which the cellulosic ethanol content was 4.2%, and the amount of the obtained cellulosic ethanol was 7.50kg. A total amount of the obtained cellulosic ethanol was 10.85kg, and a conversion rate of the cellulosic ethanol was 84.5%.

### Embodiment 7

The enzymatic effect of the method for producing cellulosic ethanol by biomass saccharification and fermentation in the present disclosure was compared with the enzymatic effect of the simultaneous saccharification and fermentation process.

Data in Embodiment 4 and Embodiment 5 was collected to calculate the ethanol conversion rate of the corn cob waste with 20% dry substrate according to the method in the present disclosure.

For the existing synchronous saccharification and fermentation process, corn cob waste with a dry substrate content of 20kg (w/w) was added to an aqueous solution containing a citric acid buffer system to make the mixture reach 100kg, a pH of the mixture was maintained at 5.0~6.0, 0.8kg cellulase Cellic CTec 2 was added to the mixture, the mixture was stirred evenly, and 1 kg of Angel yeast powder was inoculated into the mixture. Under the stirring of 300 rpm, the mixture was reacted at 35°C for 6 days to obtain 100.5 kg of material in which a content of the cellulosic ethanol was 5.2% and a conversion rate of the cellulosic ethanol was 76.9%.

Compared with the existing simultaneous saccharification and fermentation process, the conversion rate of cellulosic ethanol of the method in the present disclosure was increased from 76.9% to more than 80%, and the conversion rate improvement effect is obvious.

The above descriptions are only preferred embodiments of the present disclosure and should not be construed as the limitation of the present disclosure. Any modifications, equivalent substitutions, and improvements made within the spirit and principles of the present disclosure should be included within the scope of the present disclosure.

## Claims

1. A system for producing cellulosic ethanol by biomass saccharification and fermentation, comprising:
a reactor A configured to carry out an enzymatic hydrolysis reaction of a biomass feedstock;
a reactor B configured to carry out a fermentation reaction of the biomass feedstock; and
a collection tank configured to collect the cellulosic ethanol produced from the fermentation reaction of the biomass feedstock, wherein
an enzymatic hydrolysis dosing pipe and a fermentation dosing pipe are respectively provided on the reactor A and the reactor B,
an outlet of the reactor A is connected to an inlet of the reactor B through a fermentation pipe, a diaphragm pump A being provided on the fermentation pipe,
an outlet of the reactor B is connected to an inlet of the reactor A through a discharge pipe, a diaphragm pump B and a first discharge pipe being provided on the discharge pipe, the first discharge pipe being located behind the diaphragm pump B;
an upper part of the reactor A is connected to an inlet of the collection tank through an ethanol pipe,
an insulation layer is provided outside the reactor A for heating and insulation; and
a coil insulation device is provided at a connection of the ethanol pipe and the reactor A, the coil insulation device and the insulation layer being respectively connected to a hot water tank through a hot water pipe, a centrifugal pump being provided on the hot water pipe.

2. The system of claim 1, wherein a storage tank, a diaphragm pump C, and a second discharge pipe are provided on the discharge pipe, the storage tank being located behind the first discharge pipe and configured to store material discharged from the reactor B, the second discharge pipe being located behind the diaphragm pump C.

3. The system of claim 1 or 2, wherein an ethanol freezing device configured to condense cellulosic ethanol vapors is provided at a connection of the ethanol pipe and the collection tank.

4. The system of claim 1 or 2, wherein a fermentation cooling device configured to cool fermenting material inside the reactor B is provided outside the reactor B.

5. The system of claim 1 or 2, wherein a stirring device is respectively provided in the reactor A and in the reactor B.

6. A method for producing cellulosic ethanol by biomass saccharification fermentation, wherein the method is implemented by the system of any one of claims 1-5, and the method comprises:
in step 1, adding the biomass feedstock with 15%-20% of a dry substrate to a citric acid buffer solution with a concentration of 0.1 mol/L, adding cellulase with 4% -6% of the dry substrate, maintaining a pH of the citric acid buffer solution at 5.0~6.0, and dosing the prepared mixture to the reactor A through the enzymatic hydrolysis dosing pipe;
in step 2, maintaining a temperature of the reactor A at an enzymatic hydrolysis reaction temperature T, maintaining the pH value of material in the reactor A, and carrying out a first enzymatic hydrolysis reaction of the material in the reactor A for 10~14 hours under stirring;
in step 3, transporting the material after the enzymatic hydrolysis reaction in the reactor A to the reactor B through the fermentation pipe, adding fermentation strains with a mass ratio of 0.1% - 1% to the reactor B through the fermentation dosing pipe, maintaining a fermentation temperature at a suitable fermentation temperature for the fermentation strains, and carrying out a first fermentation reaction of the material in the reactor B for 10~14 hours under stirring;
in step 4, after completing the first fermentation reaction, transporting the material in the reactor B to the reactor A through the discharge pipe, wherein the cellulosic ethanol in the material produced by the fermentation is evaporated into cellulosic ethanol vapors in the reactor A, and the cellulosic ethanol vapors flow into the collection tank through the ethanol pipe and are condensed into the cellulosic ethanol;
in step 5, repeating the steps 2-4, wherein a same batch of material is carried out a single-circulation for 4~6 cycles respectively in the reactor A and the reactor B, and after the circulation is completed in the reactor B, the material is discharged from the system through the first discharge pipe.

7. A method for producing cellulosic ethanol by biomass saccharification fermentation, wherein the method is implemented by the system of any one of claims 2-5, and the method comprises:
in step I, adding the biomass feedstock with 15%-20% of a dry substrate to a citric acid buffer solution with a concentration of 0.1 mol/L, adding cellulase with 4% -6% of the dry substrate, maintaining a pH of the citric acid buffer solution at 5.0~6.0, and dosing the prepared mixture to the reactor A through the enzymatic hydrolysis dosing pipe;
in step II, maintaining a temperature of the reactor A at an enzymatic hydrolysis reaction temperature T, maintaining the pH value of material in the reactor A, and carrying out a first enzymatic hydrolysis reaction of the material in the reactor A for 10~14 hours under stirring;
in step III, transporting a first batch of material after the enzymatic hydrolysis reaction in the reactor A to the reactor B through the fermentation pipe, adding fermentation strains with a mass ratio of 0.1% - 1% to the reactor B through the fermentation dosing pipe, maintaining a fermentation temperature at a suitable fermentation temperature for the fermentation strains, and carrying out a first fermentation reaction of the material in the reactor B for 10~14 hours under stirring;
after the first batch of material after the enzymatic hydrolysis reaction in the reactor A has been emptied, repeating the steps I and II, dosing a second batch of material to the reactor A, and carrying out the enzymatic hydrolysis reaction of the second batch of material in the reactor A;
in step IV, after the first fermentation reaction is completed, transporting the first batch of material in the reactor B to the storage tank through the discharge pipe for temporary storage, and after emptying the first batch of material in the reactor B, repeating the step III to transport the second batch of material in the reactor A to the reactor B through the fermentation pipe for the fermentation reaction;
in step V, after emptying the second batch of material in the reactor A, transporting the first batch of material in the storage tank to the reactor A through the discharge pipe, and repeating the step II, wherein the cellulosic ethanol in the first batch of material produced by the fermentation is evaporated into cellulosic ethanol vapors in the reactor A, and the cellulosic ethanol vapors are condensed into the cellulosic ethanol and transported to the collection tank through the ethanol pipe; and
after emptying the first batch of material in the storage tank, repeating the step IV to transport the second batch of material in the reactor B to the storage tank through the discharge pipe for temporary storage;
in step VI, repeating the steps II-V, wherein the two batches of material are carried out a double-circulation, the circulation of each of the two batches of material is carried out for 4~6 cycles respectively in the reactor A and the reactor B, and after the circulations of the two batches of material are completed in the reactor B in sequence, the two batches of material are discharged from the system in sequence through the first discharge pipe.

8. The method claim 6 or 7, wherein the enzymatic hydrolysis reaction temperature T is a highest temperature at which a survival rate of the fermentation strains is above 80%.

9. The method of claim 6 or 7, wherein the enzymatic hydrolysis reaction temperature T is determined by:
preparing a test system by using the citric acid buffer solution with a concentration of 0.1 mol/L;
adding glucose with a concentration of 100 g/L and the fermentation strains with a mass ratio of 1% in the test system;
incubating the test system for 12h at 40°C, 41°C, 42°C, 43°C, 44°C, 45°C, 46°C, 47°C, 48°C, 49°C, and 50°C, respectively;
for the test system incubated at each of the 40°C, 41°C, 42°C, 43°C, 44°C, 45°C, 46°C, 47°C, 48°C, 49°C, 50°C, measuring a count of viable bacteria of the fermentation strains using a plate counting technique; and
selecting a highest temperature at which a survival rate of the viable bacteria is 80% after the 12 hours of incubation as the enzymatic hydrolysis reaction temperature T.

10. The method of claim 6 or 7, wherein the cellulase in the enzymatic hydrolysis reaction is Cellic CTec 2.
